# EUROPÄISCHE PATENTANMELDUNG

(11) **EP 1 829 859 A2**
(43) Veröffentlichungstag der Anmeldung: **05.09.2007**
(21) Anmeldenummer: 07003721.3
(22) Anmeldetag: 23.02.2007
(51) Int. Cl.: C07C 309/17, B01F 17/00, C09D 7/12

(54) **Salze von Alkylestern sulfonierter Dicarbonsäuren**

(30) Priorität: 03.03.2006 DE 102006009971
(71) Anmelder: Cognis IP Management GmbH, 40589 Düsseldorf (DE)
(72) Erfinder: Meyer, Joachim, 40721 Hilden (DE); Van der Koelen, Thomas, 46045 Oberhausen (DE); Held, Uwe, 42553 Velbert (DE); Busch, Stefan, 46047 Oberhausen (DE)

(57) **Zusammenfassung**

Vorgeschlagen werden Salze von Mono- und Dialkylestern sulfonierter Dicarbonsäuren, wobei die Dicarbonsäuren 4 bis 8-C-Atome aufweisen und es sich bei den Alkylgruppen um solche handelt, die sich vom 2-Propylheptanol ableiten, sowie ihre Verwendung als Emulgatoren, Dispergatoren, Additive für Lacke und Additive in kosmetischen und pharmazeutischen Zusammensetzungen.

## Beschreibung

### Gebiet der Erfindung

Die Erfindung betrifft Salze spezieller Mono- und Dialkylester sulfonierter Dicarbonsäuren sowie deren Verwendung.

### Stand der Technik

Salze von Mono- bzw. Dialkylestern sulfonierter Dicarbonsäuren sind seit langem bekannt. Sie werden auch als Mono- bzw. Disulfosuccinate bezeichnet und als Tenside unter anderem in der Emulsionspolymerisation, als Netzmittel für Lacke, zur Beschichtung von Papier und Pappe sowie für pharmazeutische Zwecke eingesetzt (vergleiche z.B. den Übersichtsartikel: Breuer, W., Höfer, R.; Tenside, Surfactants, Detergents, 2003, 40 (4), 208-214.).
Aufgrund seiner guten Eigenschaften wie beispielsweise der geringen dynamischen Oberflächenspannung seiner wäßrigen Lösungen findet besonders das Natriumsalz des Di-2-ethylhexylsulfosuccinates breite Anwendung. Jedoch enthalten die entsprechenden Formulierungen produktionsbedingt und aufgrund von Esterspaltungsreaktionen freies, das heißt ungebundenes 2-Ethylhexanol. Dies ist wegen seines unangenehmen Geruches und Geschmacks für viele Anwendungen - besonders im Lebensmittelbereich - von Nachteil. Zudem sind sowohl 2-Ethylhexylalkohol als auch das Natriumsalz des Di-2-ethylhexylsulfosuccinates als wassergefährdend (Wassergefährdungsklasse 2) eingestuft.

### Beschreibung der Erfindung

Aufgabe der vorliegenden Erfindung war es, Salze von Mono- und/oder Dialkylestern sulfonierter Dicarbonsäuren - insbesondere Sulfosuccinate - bereitzustellen, die die geschilderten Nachteile von Di-2-ethylhexylsulfosuccinaten (Di-2EH-Sulfosuccinate) vermeiden. Insbesondere sollten Sulfosuccinate entwickelt werden, die kein freies 2-Ethylhexanol enthalten. Eine weitere Aufgabe bestand darin, Sulfosuccinate zu entwickeln, die sich als Additive für Lacke eignen und sich insbesondere durch eine gegenüber Di-2EH-Sulfosuccinaten verbesserte Wasserbeständigkeit der entsprechenden Lacke auszeichnen.

Gegenstand der vorliegenden Erfindung sind zunächst Salze von Mono- und/oder Dialkylestem sulfonierter Dicarbonsäuren, wobei die Dicarbonsäuren 4 bis 8-C-Atome aufweisen und es sich bei den Alkylgruppen um solche handelt, die sich vom 2-Propylheptanol ableiten.

### 2-Propylheptanol

Unter "2-Propylheptanol" wird im Rahmen der vorliegenden Erfindung C₅H₁₁CH(C₃H₇)CH₂OH verstanden, wobei der Rest C₅H₁₁ die Bedeutung n-C₅H₁₁, C₂H₅CH(CH₃)CH₂ oder CH₃CH(CH₃)CH₂CH₂ haben kann. Das den Sulfosuccinaten der vorliegenden Erfindung zu Grunde liegende 2-Propylheptanol kann in Form jeder der gerade genannten drei Species oder beliebiger Mischungen dieser Species eingesetzt werden.

2-Propylheptanol kann auf verschiedenen Wegen hergestellt werden, etwa ausgehend von Valeraldehyd durch Aldolkondensation und nachfolgende Hydrierung. Des weiteren kann 2-Propylheptanol durch Kondensation von 1-Pentanol (als Mischung der entsprechenden Methylbutanole) in Gegenwart von KOH bei erhöhten Temperaturen im Sinne einer Guerbet-Reaktion hergestellt werden. Schließlich ist 2-Propylheptanol durch Hydroformylierung von Butenen nebst anschließender Aldolkondensation zugänglich.

In einer Ausführungsform setzt man ein 2-Propylheptanol zur Herstellung der erfindungsgemäßen Sulfosuccinate ein, dessen Gehalt an n-C₅H₁₁CH(C₃H₇)CH₂OH mindestens 98% beträgt. Substanzen dieser Reinheit werden vorzugsweise durch Guerbet-Reaktion von 1-Pentanol hergestellt.

In einer weiteren Ausführungsform setzt man ein 2-Propylheptanol zur Herstellung der erfindungsgemäßen Sulfosuccinate ein, das in folgender kommerziell erhältlichen Angebotsform in Form eines Isomerengemisches von
a) 70-99% n-C₅H₁₁CH(C₃H₇)CH₂OH und
b) 1-30% eines Gemisches der Alkohole C₂H₅CH(CH₃)CH₂CH(C₃H₇)CH₂OH und CH₃CH(CH₃)CH₂CH₂CH(C₃H₇)CH₂OH
vorliegt.

Die sich vom 2-Propylheptanol ableitenden Alkylreste n-C₅H₁₁CH(C₃H₇)CH₂, C₂H₅CH(CH₃)CH₂CH(C₃H₇)CH₂ und CH₃CH(CH₃)CH₂CH₂CH(C₃H₇)CH₂ werden nachfolgend auch abkürzend als 2PH bezeichnet.

### Salze von Mono- und/oder Dialkylestern sulfonierter Dicarbonsäuren

Die erfindungsgemäßen Salze von Mono- und/oder Dialkylestern sulfonierter Dicarbonsäuren leiten sich von Dicarbonsäuren mit 4 bis 8 C-Atomen ab. Ihre Herstellung kann nach allen dem Fachmann einschlägig bekannten Methoden erfolgen.

Beispiele für geeignete Dicarbonsäuren sind Maleinsäure, Fumarsäure und Bernsteinsäure.

In einer bevorzugten Ausführungsform handelt es sich bei den erfindungsgemäßen Verbindungen um Substanzen der Formel (I)

R¹O₂C-CH₂-CH(SO₃M¹)-CO₂R² (I)

wobei M' ein Kation ist und die Reste R¹ und R² unabhängig voneinander M' oder eine Alkylgruppe n-C₅H₁₁CH(C₃H₇)CH₂, C₂H₅CH(CH₃)CH₂CH(C₃H₇)CH₂OH oder CH₃CH(CH₃)CH₂CH₂CH(C₃H₇)CH₂OH bedeuten, mit der Maßgabe, dass höchstens einer der Reste R¹ und R² von die Bedeutung M¹ haben darf.

Besonders bevorzugt sind die Dialkylsulfosuccinate, also Verbindungen der Formel (II)

R¹O₂C-CH₂-CH(SO₃M¹)-CO₂R² (II)

wobei M' ein Kation ist und die Reste R¹ und R² unabhängig voneinander ausgewählt sind aus der Gruppe n-C₅H₁₁CH(C₃H₇)CH₂, C₂H₅CH(CH₃)CH₂CH(C₃H₇)CH₂OH und CH₃CH(CH₃)CH₂CH₂CH(C₃H₇)CH₂OH.

Bevorzugte Kationen der erfindungsgemäßen Salze von Mono- und/oder Dialkylestern sulfonierter Dicarbonsäuren sind die Alkalikationen Natrium und Kalium sowie das Ammoniumkation.

Die erfindungsgemäßen Salze von Mono- und/oder Dialkylestern sulfonierter Dicarbonsäuren sind geruchsneutral. Die Di-2PH-Sulfosuccinat sind wie 2-Propylheptanol nur als schwach wassergefährdend (WGK 1) einzustufen.

### Abmischungen

In einer Angebotsform liegen die erfindungsgemäßen Salze von Mono- und/oder Dialkylestem sulfonierter Dicarbonsäuren in Wasser vor.
Derartige wäßrige Lösungen weisen eine ausgezeichnete dynamische Oberflächenspannung sowie ein besonders günstiges Schaumverhalten (geringe Schaumneigung) auf.

Um bei Raumtemperatur (20 °C) flüssige Produkte zu erhalten, können wässrigen Lösungen der erfindungsgemäßen Salze von Mono- und/oder Dialkylestern sulfonierter Dicarbonsäuren bei Bedarf Co-Solvenzien zugesetzt werden. Beispiele hierfür sind ethoxylierte und/oder propoxylierte Alkohole, Butyldiglykol, Butyltriglykol oder kurzkettige Alkohole wie Ethanol, Propanol oder Butanol.

Ein weiterer Erfindungsgegenstand sind Zusammensetzungen enthaltend
(i) ein oder mehrere der erfindungsgemäßen Salze von Mono- und/oder Dialkylestern sulfonierter Dicarbonsäuren und
(ii) ein oder mehrere bei 20°C flüssige organisches Lösungsmittel mit einem Siedepunkt oberhalb 250°C bei 1 bar.
Vorzugsweise ist das Lösungsmittel (ii) ein nichtionisches Tensid, insbesondere ein Alkoholethoxylat. Beispiele geeigneter Alkohole sind Fettalkohole und/oder Oxoalkohole 10 - 18, insbesondere 12 bis 15 C-Atomen. Der Ethoxylierungsgrad beträgt vorzugsweise mit 3 bis 20 und insbesondere 5 bis 15.
Die Zusammensetzung kann neben den genannten Komponenten (i) und (ii) weitere Bestandteile enthalten. In Betracht kommen hier insbesondere kurzkettige Alkohole und/oder Wasser als Verdünnungsmittel bzw. Hilfslösemittel.

### Verwendungen

Die erfindungsgemäßen Substanzen sowie deren wässrige Lösungen und oben genannte Abmischungen lassen sich als Emulgatoren und oder Dispergatoren für die unterschiedlichsten Verwendungszwecke einsetzen.

Ein weiterer Erfindungsgegenstand ist die Verwendung der erfindungsgemäßen Substanzen sowie deren wässrige Lösungen und oben genannte Abmischungen als Emulgatoren für die Emulsionspolymerisation.

Ein weiterer Erfindungsgegenstand ist die Verwendung der erfindungsgemäßen Substanzen sowie deren wässrige Lösungen und oben genannte Abmischungen als schaumarme Netzmittel für die Emulsionspolymerisation.

Ein weiterer Erfindungsgegenstand betrifft wässrige Polymerdispersionen erhältlich durch Emulsionspolymerisation unter Einsatz der erfindungsgemäßen Substanzen sowie deren wässrige Lösungen als Emulgatoren und/oder Netzmittel und/oder Hilfsmittel zur Nachstabilisierung.

Ein weiterer Erfindungsgegenstand ist die Verwendung der erfindungsgemäßen Substanzen sowie deren wässrige Lösungen und oben genannte Abmischungen als Additive in kosmetischen und/oder pharmazeutischen Zusammensetzungen sowie in zur Ernährung geeigneten Zusammensetzungen (d.h. Zusammensetzung, die der menschlichen und/oder tierischen Ernährung dienen); dabei kann das Additiv unterschiedliche Funktionen erfüllen, insbesondere kann es als Netzmittel, Emulgator oder Dispergator wirken.

Überraschend wurde auch gefunden, daß sich mit den erfindungsgemäßen Substanzen, insbesondere den Salzen des Di-2PH-Sulfosuccinats Lacke herstellen lassen, die nach Trocknung eine deutlich bessere Wasserbeständigkeit aufweisen als entsprechende Lacke auf Basis von Di-2EH-Sulfosuccinaten. Dementsprechend ist ein weiterer Erfindungsgegenstand die Verwendung der erfindungsgemäßen Substanzen sowie deren wässrige Lösungen und oben genannte Abmischungen als Lackadditive.

### Beispiele

### Eingesetzte Substanzen

Di-(2-ethylhexyl)-maleinat wurde von Fa. Celanese, Natriumdisulfit und 2-Propylheptanol von Fa. BASF, Maleinsäureanhydrid von Fa. Sasol-Huntsman und NeoPac E-106 (PU-Acrylat-Dispersion) von Fa. DSM bezogen.
Hydropalat 875 (anionisches Netzmittel), DSX 1514 (Polyurethanpolymer) und Dehydran 1293 (Lösung eines modifizierten Polydimethylsiloxan) sind Produkte der Fa. Cognis.

### Herstellbeispiele

### Beispiel 1 (Vergleich): Herstellung von Natrium-di-(2-ethylhexyl)-sulfosuccinat (Lösung in Wasser/Ethanol)

In einen 1 1-Vierhalskolben mit mechanischem Rührer, Heizung, Rückflußkühler und Stickstoffeinleitung wurden 556 g (1,63 mol) Di-(2-ethylhexyl)-maleinat, 40 g Hydropalat 875, 160 g (0,84 mol) Natriumdisulfit und 194 g vollentsalztes Wasser vorgelegt und der Ansatz bei 104 °C unter Rückfluß und leichtem Stickstoffstrom so lange gerührt, bis eine klare Lösung entstanden war (ca. 3 Stunden). Es wurde noch 10 Minuten nachgerührt. Im Produkt war kein Sulfit mehr nachweisbar. Beim Abkühlen vergelte/verfestigte sich das Produkt, was durch Zugabe eines Co-Solvens vermieden werden konnte: Hierzu wurden bei ca. 80 °C 50 g Ethanol zugesetzt.
Die so erhaltene Lösung erhielt das Sulfosuccinat zu 75 Gew.% und wies folgende Kennzahlen auf: Epton: 16,39 %; Trockenrückenrückstand: 74,92%

### Beispiel 2 (erfindungsgemäß): Herstellung von Di-(2-propylheptyl)-maleinat

In einem 2 1-Vierhalskolben mit mechanischem Rührer, Heizung, Destillationsapparatur und Stickstoff/Vakuumanschluß wurden unter Stickstoffatmosphäre 392 g (4,0 mol) Maleinsäureanhydrid, 1330 g (8,4 mol) 2-Propylheptanol und 8,6 g (0,05 mol) p-Toluolsulfonsäure-Monohydrat eingewogen und langsam auf 140 °C erhitzt. Das entstehende Reaktionswasser wurde destillativ abgetrennt. Sobald nur noch wenig Destillat überging (etwa 3,5 Stunden nach Reaktionsbeginn) wurde der Druck langsam auf bis zu 10 mbar gesenkt und 3 Stunden gehalten. Das erhaltene Produkt wies eine Restsäurezahl von 1,66 mg KOH/g auf.

### Beispiel 3 (erfindgsgemäß): Herstellung von Natrium-di-(2-propylheptyl)-sulfosuccinat (Lösung in Wasser/Ethanol)

In einen 1 1-Vierhalskolben mit mechanischem Rührer, Heizung, Rückflußkühler und Stickstoffeinleitung wurden 574 g (1,45 mol) Di-(2-propylheptyl)-maleinat (hergestellt gemäß Beispiel 2), 40 g einer Lösung* aus (a) 70% Di-(2-propylheptyl)-sulfosuccinat, (b) 20% Wasser und (c) 10% Ethanol, (Hinweis: Die vorgenannte Lösung* aus den drei Komponenten (a), (b) und (c) wird als Produkt der beschriebenen Synthese gemäß Beispiel 3 erhalten. Bei der erstmaligen Durchführung des Beispiels 3 stand die Mischung der Komponenten (a), (b) und (c) noch nicht zur Verfügung - hier wurden stattdessen 40,0 g Hydropalat 875 eingesetzt) 142 g (0,75 mol) Natriumdisulfit und 194 g vollentsalztes Wasser vorgelegt und der Ansatz bei 104 °C unter Rückfluß und leichtem Stickstoffstrom so lange gerührt, bis eine klare Lösung entstanden war (ca. 3 Stunden). Es wurde noch 10 Minuten nachgerührt. Im Produkt war kein Sulfit mehr nachweisbar. Beim Abkühlen vergelte/verfestigte sich das Produkt, was durch Zugabe eines Co-Solvens vermieden werden konnte: Hierzu wurden bei ca. 80 °C 50 g Ethanol zugesetzt.
Das so erhaltene Produkt wies folgende Kennzahlen auf: Säurezahl: 0,18 mg KOH/g; Epton: 14,01 %; Trockenrückstand: 71,66%; Gehalt Natriumsulfat: 0,32 %.

### Anwendungsbeispiele

### Beispiel 4: Messung der dynamischen Oberflächenspannung

Die gemäß den Beispielen 1 und 3 erhaltenen Lösungen wurden mit Wasser auf eine Konzentration von jeweils 0,1 Gew.% an Di-2-Ethylhexyl-Sulfosuccinat, Na-Salz (Di-2-EH-SUS) bzw. Di-2-Propylheptyl-Sulfosuccinat, Na-Salz (Di-2-PH-SUS) verdünnt. Von diesen Lösungen wurden mittels eines Blasentensiometers der Fa. Krüss die dynamischen Oberflächenspannung bei verschiedenen Blasenfrequenzen bestimmt. Die Ergebnisse können Tabelle 1 entnommen werden.

**Tabelle 1**

| **Blasenfrequenz [Hz]** | **dyn. Oberflächenspannung [mN/m]** | |
|---|---|---|
| | **Di-2-EH-SUS (Vergleich)** | **Di-2-PH-SUS (erfindungsmäß)** |
| 0,1 | 33,8 | 26,1 |
| 0,5 | 36,1 | 26,9 |
| 1,0 | 37,2 | 28,3 |
| 5 | 39,5 | 41,5 |
| 10 | 40,6 | 50,9 |

### Beispiel 5: Wasserbeständigkeit von Lacken

Die Bestimmung der Wasserstabilität erfolgte nach DIN 68861. Hierzu wurde zunächste ein Basislack hergestellt und zwar wie folgt:
Zur Herstellung 100 g des Basislackes wurden 40,0 g NeoPac E-106 und 0,3 g DSX 1514 vorgelegt und anschließend bei laufendem Dissolver der Reihe nach 2,2 g Butylglykol, 2,2 g Methyldiglykol, 1,0 g Dehydran 1293, 53,6 g NeoPac E-106 und 0,7 g entsalztes Wasser zugegeben.
Anschließend wurde der Basislack mit 0,7 Gew.% der zu prüfenden Sulfosuccinatlösung versehen und die erhaltende Formulierung mit einem Rakel auf eine Glasplatte aufgetragen (Schichtdicke 150 µm). Nach 7-tägigier Trocknung bei 20 °C wurden Wassertropfen aufgebracht. Diese Wassertropfen ließ man 8 Stunden einwirken. Anschließend bewertete man die Veränderung der Lackoberfläche nach DIN 53230.

Die Ergebnisse können Tabelle 2 entnommen werden. Die den Angaben zur Wasserbeständigekeit in Tabelle 2 zu Grunde liegende Skala umfasst die Werte von 0 bis 5 (quasi ein "Schulnotensystem"), wobei 0 für "keine Veränderung" und 5 für "starke Veränderung" steht.

**Tabelle 2**

| **Lack** | **Wasserbeständigkeit Bewertung nach DIN 53230** |
|---|---|
| **Basislack + 0,7 Gew.% Di-2-EH-SUS-Lösung (Beispiel 1)** | **3** |
| **Basislack + 0,7 Gew.% Di-2-PH-SUS-Lösung (Beispiel 3)** | **1** |

Man erkennt ohne weiteres, dass der Lack auf Basis des erfindungsgemäßen Beispiels (also Di-2-PH-SUS enthaltend) eine deutlich bessere Wasserbeständigkeit aufweist, als der Lack auf Basis des Vergleichsbeispiels (also Di-2-EH-SUS enthaltend).

## Patentansprüche

1. Salze von Mono- und/oder Dialkylestern sulfonierter Dicarbonsäuren, **dadurch gekennzeichnet, dass** die Dicarbonsäuren 4 bis 8-C-Atome aufweisen und es sich bei den Alkylgruppen um solche handelt, die sich vom 2-Propylheptanol ableiten.

2. Verbindungen nach Anspruch 1, wobei die Dicarbonsäure-Bausteine ausgewählt werden aus der Gruppe Maleinsäure, Fumarsäure und Bernsteinsäure.

3. Verbindungen nach Anspruch 1 oder 2, wobei diese Verbindungen die Formel (I) aufweisen
R¹O₂C-CH₂-CH(SO₃M¹)-CO₂R² (I)
worin M¹ ein Kation ist und die Reste R¹ und R² unabhängig voneinander M¹ oder eine Alkylgruppe n-C₅H₁₁CH(C₃H₇)CH₂, C₂H₅CH(CH₃)CH₂CH(C₃H₇)CH₂OH oder CH₃CH(CH₃)CH₂CH₂CH(C₃H₇)CH₂OH bedeuten, mit der Maßgabe, dass höchstens einer der Reste R¹ und R² von die Bedeutung M¹ haben darf.

4. Verbindungen nach einem der Ansprüche 1 bis 3, wobei diese Verbindungen die Formel (II) aufweisen
R¹O₂C-CH₂-CH(SO₃M¹)-CO₂R² (II)
worin M' ein Kation ist und die Reste R¹ und R² unabhängig voneinander ausgewählt sind aus der Gruppe n-C₅H₁₁CH(C₃H₇)CH₂, C₂H₅CH(CH₃)CH₂CH(C₃H₇)CH₂OH und CH₃CH(CH₃)CH₂CH₂CH(C₃H₇)CH₂OH.

5. Verbindungen nach einem der Ansprüche 1 bis 4, wobei der Gehalt des 2-Propylheptanol-Bausteins an n-C₅H₁₁CH(C₃H₇)CH₂OH mindestens 98% beträgt.

6. Verbindungen nach einem der Ansprüche 1 bis 5, wobei der 2-Propylheptanol-Baustein in Form eines Isomerengemisches von
a) 70-99% n-C₅H₁₁CH(C₃H₇)CH₂OH und
b) 1-30% eines Gemisches der Alkohole C₂H₅CH(CH₃)CH₂CH(C₃H₇)CH₂OH und CH₃CH(CH₃)CH₂CH₂CH(C₃H₇)CH₂OH
vorliegt.

7. Verbindungen nach einem der Ansprüche 1 bis 6, wobei die Kationen ausgewählt werden aus der Gruppe Natrium, Kalium und Ammonium.

8. Zusammensetzungen enthaltend
(i) ein oder mehrere der erfindungsgemäßen Salze von Mono- und/oder Dialkylestem sulfonierter Dicarbonsäuren und
(ii) ein oder mehrere bei 20°C flüssige organisches Lösungsmittel mit einem Siedepunkt oberhalb 250°C bei 1 bar.

9. Zusammensetzung nach Anspruch 8, wobei man das Lösungsmittel (ii) auswählt aus der Gruppe der nichtionisches Tenside.

10. Zusammensetzung nach Anspruch 8 oder 9, wobei neben den Komponenten (i) und (ii) als weitere Bestandteile kurzkettige Alkohole und/oder Wasser als Verdünnungsmittel bzw. Hilfslösemittel enthalten sind.

11. Verwendung der Substanzen bzw. Zusammensetzungen gemäß den Ansprüchen 1 bis 9 als Emulgatoren und/oder Dispergatoren.

12. Verwendung der Substanzen bzw. Zusammensetzungen gemäß den Ansprüchen 1 bis 9 als Emulgatoren für die Emulsionspolymerisation.

13. Verwendung der Substanzen bzw. Zusammensetzungen gemäß den Ansprüchen 1 bis 9 als schaumarme Netzmittel für die Emulsionspolymerisation.

14. Wässrige Polymerdispersionen erhältlich durch Emulsionspolymerisation unter Einsatz der Substanzen bzw. Zusammensetzungen gemäß den Ansprüchen 1 bis 9 als Emulgatoren und/oder Netzmittel und/oder Hilfsmittel zur Nachstabilisierung.

15. Verwendung der Substanzen bzw. Zusammensetzungen gemäß den Ansprüchen 1 bis 9 als Additive für Lacke.

16. Verwendung der Substanzen bzw. Zusammensetzungen gemäß den Ansprüchen 1 bis 9 als Additive in kosmetischen und/oder pharmazeutischen Zusammensetzungen sowie in zur Ernährung geeigneten Zusammensetzungen.
